# EUROPEAN PATENT APPLICATION

(11) **EP 3 712 246 A1**
(43) Date of publication of application: **23.09.2020**
(21) Application number: 17932406.6
(22) Date of filing: 26.12.2017
(51) Int. Cl.: C12N 1/00, C12R 1/01, A61K 35/74, A61P 35/00, A61P 37/00

(54) **COMPOSITION FOR ENHANCING IMMUNE FUNCTION OF T CELLS AND PREPARATION METHOD THEREFOR**

(30) Priority: 17.11.2017 CN 201711144522
(71) Applicant: Shenzhen Yueyao Life Technology Co., Ltd., Nanshan District Shenzhen Guangdong 518054 (CN)
(72) Inventor: ZENG, Gucheng, Guangzhou City Guangdong 510275 (CN)
(74) Representative: Skaaning, Steffen
(86) International application number: PCT/CN2017/118590
(87) International publication number: WO 2019/095508

(57) **Abstract**

Provided is a composition comprising *Bacteroides fragilis.* The composition is used for enhancing the immune function of the T cells in a body, or for preventing and/or treating tumors.

## Description

### FIELD OF TECHNOLOGY

The present disclosure relates to the technical field of biomedicine, particularly, it relates to a composition for enhancing the immune function of the T cells, a composition for preventing and/or treating tumors, and methods for preparing the compositions.

### BACKGROUND

Malignant tumor has become the "first killer"threatening human health and life, and the death cases caused by the malignant tumor have ranked the top. Every year, about 8.7 million people suffer from malignant tumor in the world, and about 6.9 million people die from that. About1.05 million people died from late malignant tumorin China alone. Currently, limited methods can be applied to treat malignant tumors.

*Bacteroides fragilis* (*B. fragilis,* for short) is anobligate anaerobic bacterium which is Gram-negative, rod-shaped, and non-motile, having obtuse and hyperchromatic ends as well as a capsule, without spores. The *Bacteroides fragilis* can be classified into an enterotoxigenic type and anon-enterotoxigenic type. As a part of the normal intestinal flora of humans and animals, *Bacteroides fragilis* mainly existsin the colon, and besides, it can also colonize and grow in the respiratory tract, the gastrointestinal tract and the urogenital tract. Numerous researches have shown that *Bacteroides fragilis* has a good effect on the prevention and treatment of acute and chronic enteritis, dysbacteriosis, upper respiratory infection and neurosis, etc.

### SUMMARY

It is an objective of the present disclosure to provide a composition for enhancing the immune function of T cells in a body as well as preventing and/or treating a tumor.

It is another objective of the present disclosure to provide preparation methods of the composition.

Inorder to achieve the above objectives, the present disclosure discloses a use of *Bacteroides fragilis* in enhancing the immune function of T cells in a body.

The present disclosure also discloses a use of *Bacteroides fragilis* in preventing and/or treating a tumor.

The present disclosure also discloses a use of *Bacteroides fragilis* in preparing medicaments for enhancing the immune function of the T cells in a body.

The present disclosure also discloses a use of *Bacteroides fragilis* in preparing medicaments for preventing and/or treating a tumor.

The present disclosure also provides a composition for enhancing the immune function of T cells in a body, and the composition comprises *Bacteroides fragilis.*

Preferably, the *Bacteroides fragilis* is inactivated, attenuated, low-infectious or non-infectious, and it contains bacterial protein ingredient in natural structures, which can effectively decrease the expression of exhausted molecule on the T cells, thereby enhancing the immune function of the T cells.The *Bacteroides fragilis* is any one or more of the following: living *Bacteroides fragilis;* inactivated, genetically recombined, altered or modified, attenuated, chemically treated, physically treated or inactivated *Bacteroides fragilis; Bacteroides fragilis* lysate; and/or *Bacteroides fragilis* culture supernatant.

Preferably, the *Bacteroides fragilis* is inactivated by any one or more of methods of dry heating, moist heating, filtration, organic solvent, chemical reagent, ultraviolet ray, infrared ray, fermentation, freeze-drying, genetic recombination, genetic modification and genetic alternation.

The composition includes but is not limited to any one of pharmaceutical compositions, foods, health products, or food additives.

The present disclosure also provides a preparation method of composition for enhancing the immune function of T cells in a body, comprising steps of:
(1) collecting living *Bacteroides fragilis* cultures;
(2) washing the *Bacteroides fragilis* with 15% of glycerol/saline solution;
(3) resuspending the *Bacteroides fragilis* in the 15% of glycerol/saline solution;
(4) heating the *Bacteroides fragilis* for 30 minutes by 70 °C water bath to obtain inactivated *Bacteroides fragilis.*

The 15% of glycerol/saline solution refers to the saline solution containing 15% of glycerol (volume percentage).

The present disclosure also provides a composition for preventing and/or treating a tumor, wherein the composition comprises *Bacteroides fragilis.*

The tumor preferably comprises, but is not limited to, one or more of breast cancer, colorectal cancer, ovarian cancer, pancreatic cancer, prostatic cancer, carcinoma of urinary bladder, melanoma, lung cancer, stomach cancer, liver cancer, cancer of biliary duct, glioma, squamous cell carcinoma of the head or neck, cervical cancer, renal cell carcinoma, thyroid cancer, acute myeloid leukemia, myeloma, esophagus cancer, lymphoma, and skin cancer.

Preferably, the *Bacteroides fragilis* is inactivated, attenuated, low-infectious or non-infectious, and it contains bacterial protein ingredient in natural structures.

Preferably, the *Bacteroides fragilis* is inactivated by any one or more of methods of dry heating, moist heating, filtration, organic solvent, chemical reagent, ultraviolet ray, infrared ray, fermentation, freeze-drying, genetic recombination, genetic modification and genetic alternation.

The composition includes but is not limited to any one of pharmaceutical compositions, foods, health products, or food additives.

The present disclosure also provides a method of enhancing the immune function of the T cells in a body, comprising steps of:
(1) providing the composition containing inactivated or non-infectious *Bacteroides fragilis;*
(2) administrating effective dosage of the composition to the patients.

The present disclosure also provides a method for preventing and/or treating tumors, the method comprising administrating a composition containing inactivated, low- or non-infectious *Bacteroides fragilis* to the patients, to decrease the expression of inhibitory molecule Tim-3 on the T cells and enhancing the immune function of the T cells.

The present disclosure also provides a preparation method of a composition for preventing and/or treating a tumor, comprising steps of:
(1) collecting living *Bacteroides fragilis* cultures;
(2) washing the *Bacteroides fragilis* with 15% of glycerol/saline solution;
(3) resuspending the *Bacteroides fragilis* in the 15% of glycerol/saline solution;
(4) heating the *Bacteroides fragilis* for 30 minutes by 70 °C water bath to obtain inactivated *Bacteroides fragilis.*

The experimental results show that *Bacteroides fragilis* with improved effectivity may have 40%, or more, or even 40-90% better effect of decreasing or controlling the tumor growth compared with the saline control group or the non-inactivated intestinal bacteria control group.

*Bacteroides fragilis* with improved effectivity can decrease expression of function inhibitory molecule on immune cells, especially on T cells, which is also called T-cell exhausted molecule, comprising but is not limited to T-cell immunoglobulin and mucin-domain containing-3 (Tim-3 or Tim 3, for short). Compared with the saline control group or the non-inactivated intestinal bacteria control group, the *Bacteroides fragilis* with improved effectivity decreases expression of the T-cell exhausted molecule, such as Tim-3 by 35-65%, or more.

*Bacteroides fragilis* with improved effectivity can simultaneously decrease expression of the T-cell exhausted molecules on CD4+T and CD8+T cells which are the most important two types of antitumor immune cells, thus to enhance the antitumor immune function of CD4+T and CD8+T cells, wherein the T-cell exhausted molecules on CD4+T and CD8+T cells comprises but is not limited to Tim-3.

### BRIEF DESCRIPTION

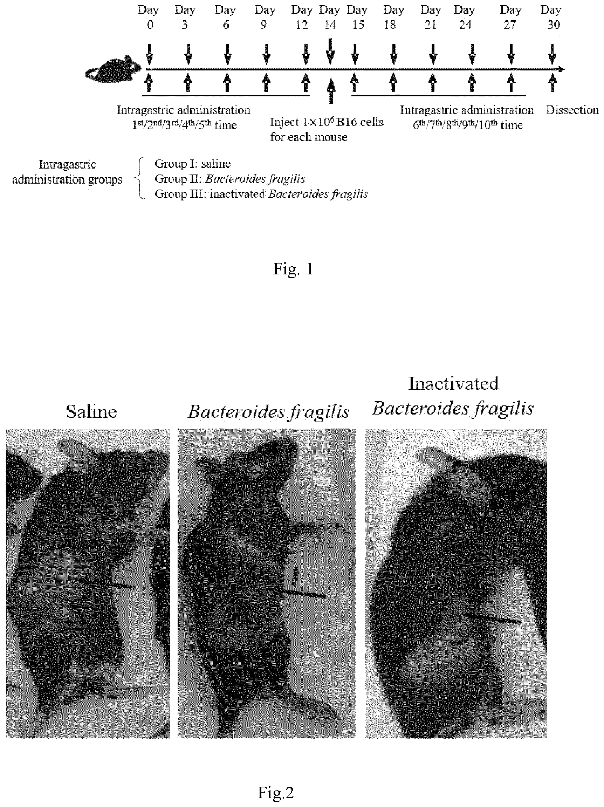

Fig. 1 is a schematic flow chart of an experiment for detecting the therapeutic and/or preventive effect of *Bacteroides fragilis* and inactivated *Bacteroides fragilis* on melanoma in mice.
Fig. 2 shows an in-situ detectionof inhibiting effect of *Bacteroides fragilis* and inactivated *Bacteroides fragilis* on the tumor growth in mice, wherein the dotted-line circle indicates the location and volumeof the tumor. The bigger the diameter of the circle is, the faster the tumor grows and the larger the tumor volume is. It can be seen from the figure that the murine tumor treated with inactivated *Bacteroides fragilis* is significantly smaller than that of the other two groups.

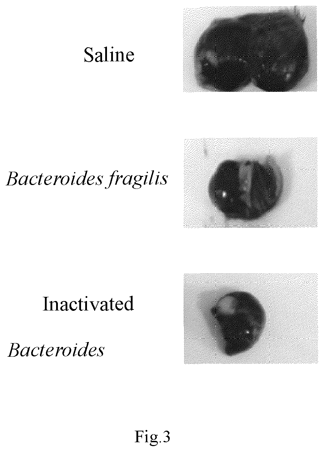

Fig. 3 is the tumor detection photograph showing the inhibiting effect of *Bacteroides fragilis* and inactivated *Bacteroides fragilis* on the tumor growth in mice. It can be seen from the figure that the murine tumor volumeis significantly decreased after a treatment of inactivated *Bacteroides fragilis,* compared with the saline control group or non-inactivated control group.

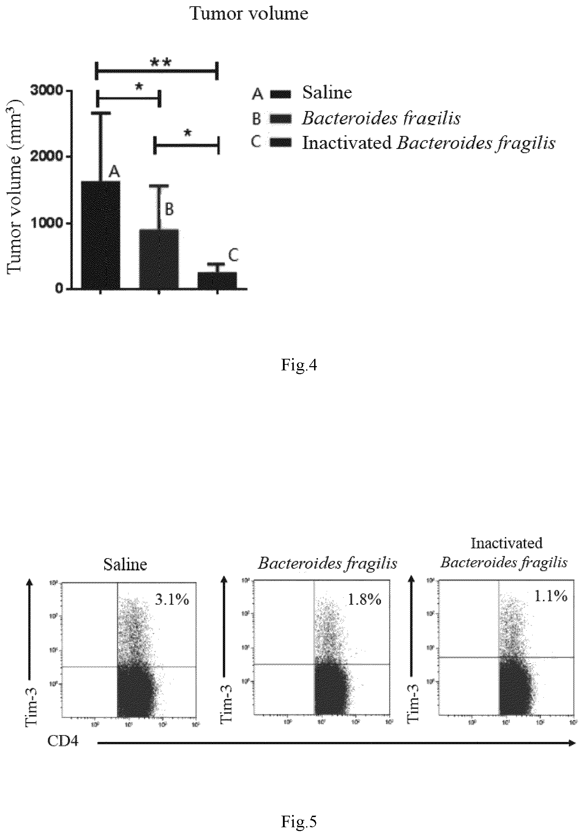

Fig.4 is a comparative statistical analysis chart of tumor volumein mice after *Bacteroides fragilis* and inactivated *Bacteroides fragilis* are used for prevention and /or treatment. Compared with the saline control group or non-inactivated control group, the inactivated *Bacteroides fragilis* has a 40-90%, or more, better effect of inhibiting the tumor growth volume. "*" represents student *t*-test *p*<0.05, "**" represents student *t*-test *p*<0.01. Either *p*<0.05 or *p*<0.01 indicates a statistic significant difference. There are 12 mice in each group.
Fig.5 shows a flow cytometry graphofonemouse for each group, exhibiting the expression of inhibitory molecule Tim-3 on CD4+T cells after *Bacteroides fragilis* and inactivated *Bacteroides fragilis* are used for preventing and/or treating melanoma. The number in the right-top quadrant represents the percentage of Tim-3+CD4+T cells (both Tim-3 and CD4 are expressed on the T cells) in total CD4+T cells in the spleen. As seen from the flow cytometry quadrant graph, the inactivated *Bacteroides fragilis* decreases the expression of Tim-3 by 38-65% or more, compared with the saline control group or inactivated control group.

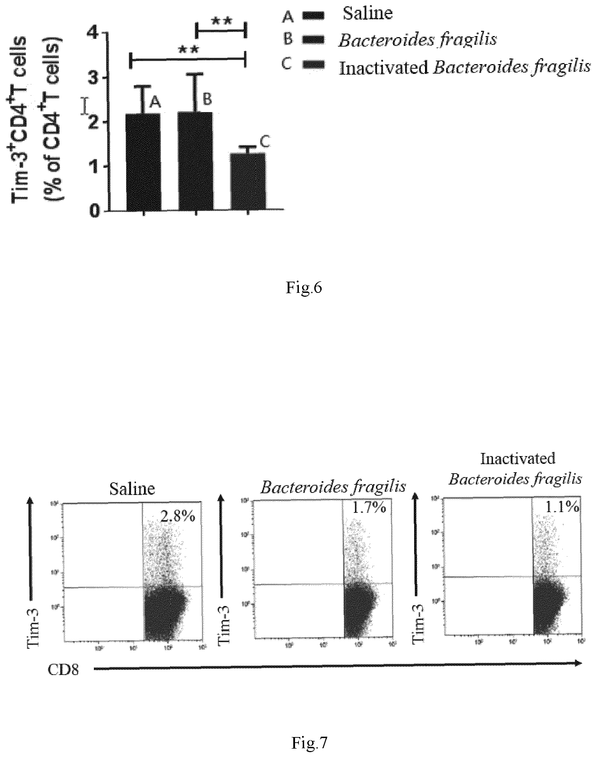

Fig.6 is a statistical analysis chart showing the expression of inhibitory molecule Tim-3 on the CD4+T cells after *Bacteroides fragilis* and inactivated *Bacteroides fragilis* are used for preventing and/or treating melanoma. As seen from the statistical chart, the inactivated *Bacteroides fragilis* significantly decrease the expression of Tim-3 on the CD4+T cells, compared with the saline control group or non-inactivated control group. In the statistical chart, "**" represents student *t*-test *p*<0.01. *p*<0.01 indicates a statistic significant difference. 12 mice in each group.
Fig.7 shows a flow cytometry graph exhibiting the expression of inhibitory molecule Tim-3 on the CD8+T cells after *Bacteroides fragilis* and inactivated *Bacteroides fragilis* are used for preventing and/or treating melanoma. The number in the right-top quadrant represents the percentage of Tim-3+CD8+T cells (both Tim-3 and CD8 are expressed on the T cells) in total CD8+T cells in the spleen. As seen from the flow cytometry quadrant graph, the inactivated *Bacteroides fragilis* decreases the expression of Tim-3 by 35-60% or more, compared with the saline control group or non-inactivated control group.

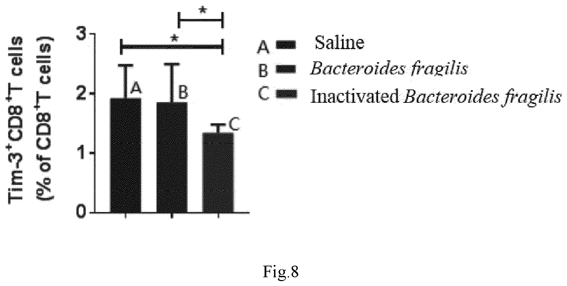

Fig.8 is a statistical analysis chart showing the expression of inhibitory molecule Tim-3 on the CD8+T cells after *Bacteroides fragilis* and inactivated *Bacteroides fragilis* are used for preventing and/or treating melanoma. As seen from the statistical chart, the inactivated *Bacteroides fragilis* significantly decrease the expression of Tim-3 on the CD8+T cells, compared with the saline control group or non-inactivated control group. In the statistical chart, "*" represents student *t*-test *p*<0.05. *p*<0.05 indicates a statistic significant difference. 12 mice in each group.

### DETAILED DESCRIPTION

The present disclosure will be further described below with reference to the embodiments without any limitation. It should be pointed out that the inactivated *Bacteroides fragilis* for enhancing the immune function of T cells in the body, and treating and/or preventing a tumor in the present disclosure, or a pharmaceutical composition, a food, a health product and a food additive containing the *Bacteroides fragilis* of the present disclosure can be applied to the indications described above and exhibits the functions described above, after they are administered to the subject. The tests have been done in all dosage forms within the scope of the present disclosure. In the following, only a small partis described in the examples just for illustration, however, it should not be construed as a limitation of the disclosure.

The *Bacteroides fragilis* is inactivated, attenuated, low-contagious or non-contagious, and can decrease the expression of exhausted cells of T-cells on CD4+T cells and/or CD8+T cells. Preferably, the *Bacteroides fragilis* is inactivated by any one or more of methods of dry heating, moist heating, filtration, organic solvent, chemical reagents, ultraviolet ray, infrared ray, fermentation, freeze-drying, genetic recombination, genetic modification and genetic alternation.

The tumor/cancer comprises but is not limited to solid tumor/cancer. In some embodiments, the tumor/cancer may be breast cancer, colorectal cancer, ovarian cancer, pancreatic cancer, prostatic cancer, carcinoma of urinary bladder, melanoma, lung cancer, stomach cancer, liver cancer, cancer of biliary duct, glioma, squamous cell carcinoma of the head or neck, cervical cancer, renal cell carcinoma, thyroid cancer, acute myeloid leukemia, myeloma, esophagus cancer, lymphoma, skin cancer, or any combination thereof.

### Example 1 Bacteroides fragilis culture

### Culture method

Step 1: A lyophilized preserved *Bacteroides fragilis* strain (purchased from ATCC official website) was taken, then 200µL Tryptic Soy Broth (TSB) was added to reconstitute it. Bacterial solution was streaked on the blood agar plate. Then the plate was placed in a biochemical incubator after an air exhaust processing by an anaerobic jar gassing system, and cultured in anaerobic environment at 37°C for 48h;
Step 2: Monoclonal colony was selected and inoculated in 10mL TSB, followed by being cultured in anaerobic environment at 37°C for 12h;
Step 3:1% (v/v) of strain was inoculated in 500mL TSB in a flask and cultured in anaerobic environment at 37°C for 48 hours.
Step 4: The bacterial solution was collected and centrifuged at 6000 rpm for 10 min. The bacterial sludge was washed twice with saline, and was finally reconstituted with saline for later use. The viable bacteria were counted.

### Example 2 Experiment of the therapeutic effect of Bacteroides fragilis on tumors (melanoma) in mice.

Fig. 1 is a schematic flow chart of an experiment for detecting the therapeutic and/or preventive effect of *Bacteroides fragilis* and inactivated *Bacteroides fragilis* on tumors in mice (melanoma).

### 1. Culture method

The culture method of *Bacteroides fragilis* is the same as that in Example 1.

### 2. Sample preparation

### 1) Preparation of living Bacteroides fragilis ZY-312

Step 1: A lyophilized preserved strain (purchased from ATCC official website) was taken, and then 200µL lyophilized preserved bacterial culture medium was added to reconstitute it. Then 20µL bacterial solution was pipetted, and was streaked on the blood agar plate. The plate was placed in a biochemical incubator after an air exhaust processing by an anaerobic jar gassing system, and then was cultured in anaerobic environment at 37°C for 48h;
Step 2: Monoclonal colony was selected and inoculated in 10mL TSB, and followed by being cultured in anaerobic environment at 37°C for 12h;
Step 3: 1% (v/v) of strain was inoculated in 500 mL TSB in a flask, and cultured in anaerobic environment at 37°C for 48 hours;
Step 4: The bacterial solution was taken and centrifuged with a centrifuge at 6000 rpm for 10 minutes. The bacterial sludge was washed twice with saline, and was finally reconstituted with saline for later use. The viable bacteria were counted.

### 2) Inactivated Bacteroides fragilis

The bacterial solution was heated in a water bath at 70°C for 30 minutes to obtain inactivated bacterial solution.

Experimental animals: Thirty-six C57BL/6 mice, 3 to 4 weeks old, in good mental state, were purchased from the Experimental Animal Center of Sun Yat-sen University. The mice were randomly divided into 3 groups, 12 mice for each, and the 3 groups were the saline control group, the *Bacteroides fragilis* gavage group (living bacteria group or non-inactivated group, for short), and the inactivated *Bacteroides fragilis* gavage group (inactivated group for short), respectively.

### Experimental process and results:

As shown in Fig. 1, 1 × 10⁹ CFU of *Bacteroides fragilis,* 1 × 10⁹ CFU of inactivated *Bacteroides fragilis,* and saline control were respectively administered intragastrically to the three groups of mice for 2 weeks, and the body weights of the mice were measured daily. The murine tumor (melanoma) cells B16 were digested with TE after they were grown to logarithmic phase, and the medium was used for neutralizing. The cells were collected through centrifugation, and then washed twice with DPBS to remove the residual serum. The cells were resuspended with DPBS and counted. 1×10⁶B16 cells were inoculated subcutaneously into the right armpit of each mouse, and the intragastrical administration were continued. After 2 weeks, the tumor-bearing mice were killed to obtain the subcutaneous tumors, and tumor volume was measured.

Considering CD4+T cell and CD8+T cell are two important subgroups of T cells for anti-tumor in a body, anti-CD3, anti-CD4, anti-CD8 and anti-Tim-3 monoclonal fluorescent antibodies were used to label and separate the lymphocyte cells of spleenin mice, and a flow cytometry was used to analyze the percentage of expression of Tim-3 on CD4+T cells and CD8+T cells in the spleen of the mice.

The results show that both *Bacteroides fragilis* and its inactivated bacteria can significantly inhibit formation and growth of murine tumor (Fig. 2 and Fig. 3, the tumor is indicated by the arrow in Fig. 2). The experimental results shown in Fig. 2, Fig. 3 and Fig. 4 illustrate that the murine tumor (melanoma) in the saline control group become larger gradually over time, and the tumor in the inactivated *Bacteroides fragilis* group is significantly smaller than that in the saline control group and non-inactivated control group (there is statistically significant difference). These results (Fig. 2, Fig.3 and Fig.4) show that *Bacteroides fragilis* being activated can significantly enhance the anti-tumor effect of the body and inhibit the tumor growth, which is more effective for preventing and treating tumors (e.g. melanoma).

Tim-3 is an exhausted molecule to inhibit the anti-tumor function of T cell, and higher expression of Tim-3 indicates that the anti-tumor immune function of the T cells in the body is inhibited, low-active and/or exhausted. It is a key point to control and decrease the expression of Tim-3 on the T cells, in order to improve or enhance the anti-tumor immune function of the T cells in a body.The lower the expressionof Tim-3 is, the better the anti-tumor immune function of the T cell has. The results shown in Fig. 5, Fig. 6, Fig. 7 and Fig. 8 indicate that the inactivated *Bacteroides fragilis,* compared with the saline or non-inactivated *Bacteroides fragilis,* can inhibit the expression of Tim-3 on CD4+T cell and/or CD8+T cell more effectively and significantly (the effect of inhibiting expression of Tim-3 is statistically significant), and thus enhance the immune function of the T cells and anti-tumor immunity of the body. This result further explains why the inactivated *Bacteroides fragilis* has better anti-tumor effect, compared with saline or living *Bacteroides fragilis.*

The above content is a further detailed description of the technical solution in combination with the preferred embodiments of the present disclosure. It cannot be considered that the specific implementation of the present disclosure is limited to the above descriptions. For those skilled in the art, any simple deductions or replacements without departing from the inventive concept of the invention should all be regarded as belonging to the protection scope of the invention.

## Claims

1. A composition for enhancing immune function of T cells in a body, comprising *Bacteroides fragilis.*

2. The composition according to claim 1, wherein the *Bacteroides fragilis* is one or more selected from living *Bacteroides fragilis;* inactivated, genetically recombined, altered or modified, attenuated, chemically treated, physically treated or inactivated *Bacteroides fragilis; Bacteroides fragilis* lysate; and/or *Bacteroides fragilis* culture supernatant.

3. The composition according to claim 1, wherein the *Bacteroides fragilis* is inactivated by any one or more of methods of dry heating, moist heating, filtration, organic solvent, chemical reagent, ultraviolet ray, infrared ray, fermentation, freeze-drying, genetic recombination, genetic modification and genetic alternation.

4. The composition according to claim 1, wherein the composition is any one of pharmaceutical compositions, foods, health products, and food additives.

5. A preparation method of the composition according to claim 1, comprising steps of:
(1) collecting living *Bacteroides fragilis* cultures;
(2) washing the *Bacteroides fragilis* with 15% of glycerol/saline solution, wherein the 15% of glycerol/saline solution refers to the saline solution containing 15 vol.% glycerol;
(3) resuspending the *Bacteroides fragilis* in the 15% of glycerol/saline solution;
(4) heating the *Bacteroides fragilis* for 30 minutes by using a 70 °C water bath to obtain inactivated *Bacteroides fragilis.*

6. A composition for preventing and/or treating a tumor, comprising *Bacteroides fragilis.*

7. The composition according to claim 6, wherein the *Bacteroides fragilis* is one or more selected from living *Bacteroides fragilis;* inactivated, genetically recombined, altered or modified, attenuated, chemically treated, physically treated or inactivated *Bacteroides fragilis; Bacteroides fragilis* lysate; and/or *Bacteroides fragilis* culture supernatant.

8. The composition according to claim 6, wherein the *Bacteroides fragilisis* inactivated by any one or more of methods of dry heating, moist heating, filtration, organic solvent, chemical reagent, ultraviolet ray, infrared ray, fermentation, freeze-drying, genetic recombination, genetic modification and genetic alternation.

9. The composition according to claim 6, wherein the composition is any one of pharmaceutical compositions, foods, health products and food additives.

10. The composition according to claim 6, wherein the tumor is one or more of breast cancer, colorectal cancer, ovarian cancer, pancreatic cancer, prostatic cancer, carcinoma of urinary bladder, melanoma, lung cancer, stomach cancer, liver cancer, cancer of biliary duct, glioma, squamous cell carcinoma of the head or neck, cervical cancer, renal cell carcinoma, thyroid cancer, acute myeloid leukemia, myeloma, esophagus cancer, lymphoma, and skin cancer.

11. A preparation method of the composition according to claim 6, comprising steps of:
(1) collecting living *Bacteroides fragilis* cultures;
(2) washing the *Bacteroides fragilis* with 15% of glycerol/saline solution, wherein the 15% of glycerol/saline solution refers to the saline solution containing 15 vol.% glycerol;
(3) resuspending the *Bacteroides fragilis* in the 15% of glycerol/saline solution;
(4) heating the *Bacteroides fragilis* for 30 minutes by using a 70 °C water bath to obtain living *Bacteroides fragilis.*

12. Use of *Bacteroides fragilis* in preparation of composition for enhancing the immune function of the T cells in a body.

13. Use of *Bacteroides fragilis* in preparation of composition for preventing or treating a tumor.
